(19)

**Europäisches Patentamt**
**European Patent Office**
**Office européen des brevets**

(11) **EP 4 646 693 B1**

(12) **EUROPEAN PATENT SPECIFICATION**

(45) Date of publication and mention
of the grant of the patent:
**15.07.2026 Bulletin 2026/29**

(21) Application number: **24702432.6**

(22) Date of filing: **04.01.2024**

(51) International Patent Classification (IPC):
*G06T 7/00* (2017.01)

(52) Cooperative Patent Classification (CPC):
**G06T 7/0012;** G06T 2207/10132;
G06T 2207/20076; G06T 2207/20081;
G06T 2207/20084; G06T 2207/30068;
G06T 2207/30096

(86) International application number:
**PCT/IT2024/050001**

(87) International publication number:
**WO 2024/147166 (11.07.2024 Gazette 2024/28)**

(54) **SYSTEM AND METHOD FOR ESTIMATING THE METASTATIC STATUS OF A SENTINEL LYMPH NODE**

SYSTEM UND VERFAHREN ZUR SCHÄTZUNG DES METASTASIERUNGSSTATUS EINES WÄCHTERLYMPHKNOTENS

SYSTÈME ET PROCÉDÉ D'ESTIMATION DE L'ÉTAT MÉTASTATIQUE D'UN GANGLION LYMPHATIQUE SENTINELLE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **05.01.2023 IT 202300000105**

(43) Date of publication of application:
**12.11.2025 Bulletin 2025/46**

(73) Proprietor: **I.R.C.C.S Istituto Tumori "Giovanni
Paolo II"**
**70124 Bari (BA) (IT)**

(72) Inventors:
• **MASSAFRA, Raffaella**
**70124 BARI (BA) (IT)**
• **FANIZZI, Annarita**
**70124 BARI (BA) (IT)**
• **COMES, Maria Colomba**
**70124 BARI (BA) (IT)**
• **BOVE, Samantha**
**70124 BARI (BA) (IT)**

(74) Representative: **Mele, Emanuele et al**
**Barzanò & Zanardo S.p.A.**
**Via Piemonte, 26**
**00187 Roma (IT)**

(56) References cited:
• **BOVE SAMANTHA ET AL: "A ultrasound-based
radiomic approach to predict the nodal status in
clinically negative breast cancer patients", vol.
12, no. 1, 12 May 2022 (2022-05-12),
XP093064078, Retrieved from the Internet
<URL:https://www.nature.com/articles/
s41598-022-11876-4.pdf> DOI: 10.1038/
s41598-022-11876-4**
• **LEE YAN-WEI ET AL: "Axillary lymph node
metastasis status prediction of early-stage
breast cancer using convolutional neural
networks", COMPUTERS IN BIOLOGY AND
MEDICINE, vol. 130, 1 March 2021 (2021-03-01),
US, pages 104206, XP093064060, ISSN:
0010-4825, DOI: 10.1016/
j.compbiomed.2020.104206**

**(Cont. next page)**

- GÓMEZ-FLORES WILFRIDO ET AL: "A comparative study of pre-trained convolutional neural networks for semantic segmentation of breast tumors in ultrasound", COMPUTERS IN BIOLOGY AND MEDICINE, NEW YORK, NY, US, vol. 126, 8 October 2020 (2020-10-08), XP086322866, ISSN: 0010-4825, [retrieved on 20201008], DOI: 10.1016/J.COMPBIOMED.2020.104036
- ZHENG XUEYI ET AL: "Deep learning radiomics can predict axillary lymph node status in early-stage breast cancer", NATURE COMMUNICATIONS, vol. 11, no. 1, 6 March 2020 (2020-03-06), UK, XP093151602, ISSN: 2041-1723, Retrieved from the Internet <URL:https://www.nature.com/articles/s41467-020-15027-z.pdf> DOI: 10.1038/s41467-020-15027-z
- AFSHAR PARNIAN ET AL: "From Handcrafted to Deep-Learning-Based Cancer Radiomics: Challenges and opportunities", IEEE SIGNAL PROCESSING MAGAZINE, IEEE, USA, vol. 36, no. 4, 1 July 2019 (2019-07-01), pages 132 - 160, XP011732388, ISSN: 1053-5888, [retrieved on 20190626], DOI: 10.1109/MSP.2019.2900993
- SERRANO A J ET AL: "Feature selection using ROC curves on classification problems", NEURAL NETWORKS (IJCNN), THE 2010 INTERNATIONAL JOINT CONFERENCE ON, IEEE, PISCATAWAY, NJ, USA, 18 July 2010 (2010-07-18), pages 1 - 6, XP031771645, ISBN: 978-1-4244-6916-1

**Description**

The present invention relates to a system and a method for classifying metastatic status of a sentinel lymph node (SLN).

In particular, the present invention relates to a computer-implemented method that allows to classify the metastatic status of a sentinel lymph node in patients affected by breast cancer and with clinically negative lymph nodes, by analyzing exclusively the characteristics of the primary tumor.

This invention also relates to a computer program product and a system configured to classify the metastatic status of a sentinel lymph node.

**Background of the invention**

An accurate detection of the metastatic status is essential to reduce the chance of future occurrence of metastasis in patients affected by breast carcinoma.

After a first breast cancer diagnosis, if the patient has been found negative both for clinical and instrumental examination, the lymph status is commonly evaluated by performing an intraoperative procedure.

The intraoperative procedure involves biopsy of the sentinel lymph node, which is the first lymph node to be reached from a primary tumor as it is proximal to the primary tumor. The intraoperative procedure involves the use of a molecular method for the identification of metastatic cells inside the lymph nodes, such as the OSNA (One Step Nucleic Acid Amplification) method. Such intraoperative procedure is invasive and is expensive both in terms of time and in terms of costs. In addition, this procedure can induce significant side effects.

Bove, S., Comes, M.C., Fanizzi, A. & Massafra, R. "A ultrasound-based radiomic approach to predict the nodal status in clinically negative breast cancer patients" Sci Rep 12, 7914 (2022). https://doi.org/10.1038/s41598-022-11876-4 (12 May 2022) proposes a non-invasive alternative to the OSNA procedure described above.

This non-invasive procedure makes it possible to estimate the status of the sentinel lymph node by means of a machine learning model capable of predicting the lymph node status from an ultrasound image of the primary tumor.

In such a procedure, the ultrasound image of the acquired primary tumor is processed in order to obtain four different regions of interest (original ROI, intratumoral ROI, peritumoral ROI, combined ROI) previously manually identified by the radiologist, then radiomic features are extracted, i.e. a limited number of features manually extracted by the operator that describe both the tissue characteristics, but also quantitative and exemplary characteristics of the distribution of gray levels within the image.

The determination of a status of the sentinel lymph node is based on the joint analyses of the clinical variables with the radiomic features extracted from the four different regions of interest. These clinical variables and radiomic features are correlated with the metastatic nodal status. By means of this joint analysis, it is possible to determine whether the primary tumor has acquired the ability to migrate to the sentinel lymph node, or that has migrated, thus obtaining an estimate of the metastatic status of the sentinel lymph node.

Although the joint analysis of clinical variables with radiological characteristics extracted from the ROI represents an alternative applicable to clinical practice, this method is based on the operator's experience and it would therefore be beneficial to optimize such a method in order to improve accuracy and applicability to clinical practice.

LEE YAN-WEI ET AL: "Axillary lymph node metastasis status prediction of early-stage breast cancer using convolutional neural networks", COMPUTERS IN BIOLOGY AND MEDICINE, vol. 130, 1 March 2021 (2021-03-01), page 104206, describes the prediction of axillary lymph node metastasis status in early-stage breast cancer using convolutional neural networks.

AFSHAR PARNIAN ET AL: "From Handcrafted to Deep-Learning-Based Cancer Radiomics: Challenges and opportunities", IEEE SIGNAL PROCESSING MAGAZINE, IEEE, USA, vol. 36, no. 4, 1 July 2019, pages 132-160, describes aspects related to the transition from handcrafted radiomics to Deep Learning-based radiomics.

SERRANO A J ET AL: "Feature selection using ROC curves on classification problems", NEURAL NETWORKS (IJCNN), THE 2010 INTERNATIONAL JOINT CONFERENCE ON, IEEE, PISCATAWAY, NJ, USA, 18 July 2010 (2010-07-18), pages 1-6, describes feature selection using ROC curves for classification problems.

**Object of the invention**

The aim of the present invention is to overcome said disadvantages, by providing a method to classify the metastatic status of a sentinel lymph node.

Therefore, the object of the invention is a method as claimed in claim 1, a system as claimed in claim 12 and a computer program product as claimed in claim 13.

Further preferred embodiments are described in the dependent claims.

## Brief description of the drawings

The present invention will now be described, for illustrative but not limitative purposes, according to an embodiment thereof, with particular reference to the accompanying figures, in which:

FIG. 1 shows a block diagram of a method for classifying the status of a sentinel lymph node, according to an embodiment of the present invention;
FIG. 2a shows an ultrasound image of the primary tumor comprising one or more markers, according to an embodiment of the present invention;
FIG. 2b shows the ultrasound image in FIG. 1 wherein said one or more markers have been selected, according to an embodiment of the present invention;
FIG. 2c shows the ultrasound image of FIG. 1 wherein said one or more markers have been removed and the pixels of the image have been reconstructed, according to an embodiment of the present invention;
FIG. 3 shows a binary mask wherein pixels other than zero (i.e. the pixels in white) correspond to the designated region of the primary tumor; and
FIG. 4 shows a region of interest of the ultrasound image that includes both the intratumoral region and the peritumoral region of the primary tumor.

## Detailed description of the invention

In the various figures, similar parts will be indicated by the same reference numerals.

Certain steps of the method will be described below using specific instructions of the programming platform Matlab® but it is well evident that the invention should not be considered limited to the use of such a specific platform and/or instructions.

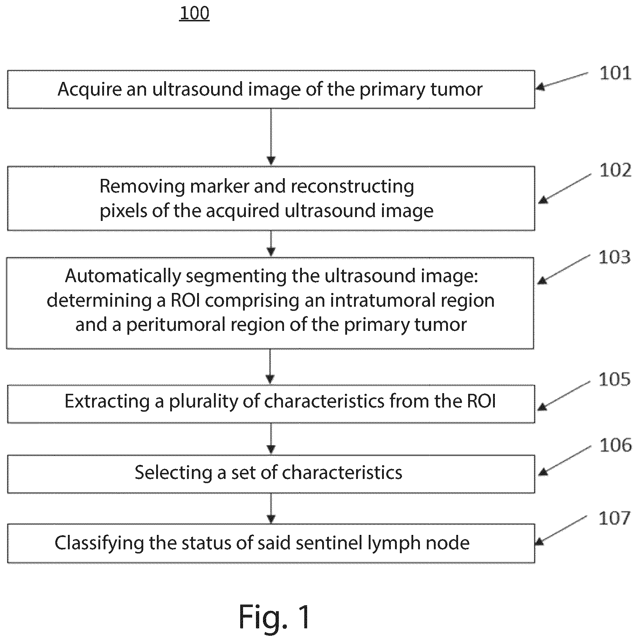

With reference to FIG. 1, a computer-implemented method 100 is shown for classifying the status of a sentinel lymph node, such as, for example, an axillary sentinel lymph node.

The method 100 makes it possible to predict, in an automatic manner, the status of the sentinel lymph node in patients affected by a breast cancer that is judged as being clinically negative, for example during the diagnostic step of the primary tumor, and on the basis of the use of an ultrasound image 1 of the primary tumor 10 that can be appropriately selected by the radiologist.

The method 100 comprises the step of acquiring 101 an ultrasound image 1 of the primary tumor 10, for example an image of a breast cancer.

The ultrasound image 1 can be obtained from an ultrasound examination by means of ultrasounds.

The image can be acquired directly by the system or it can be appropriately selected by the radiologist before being acquired by the system.

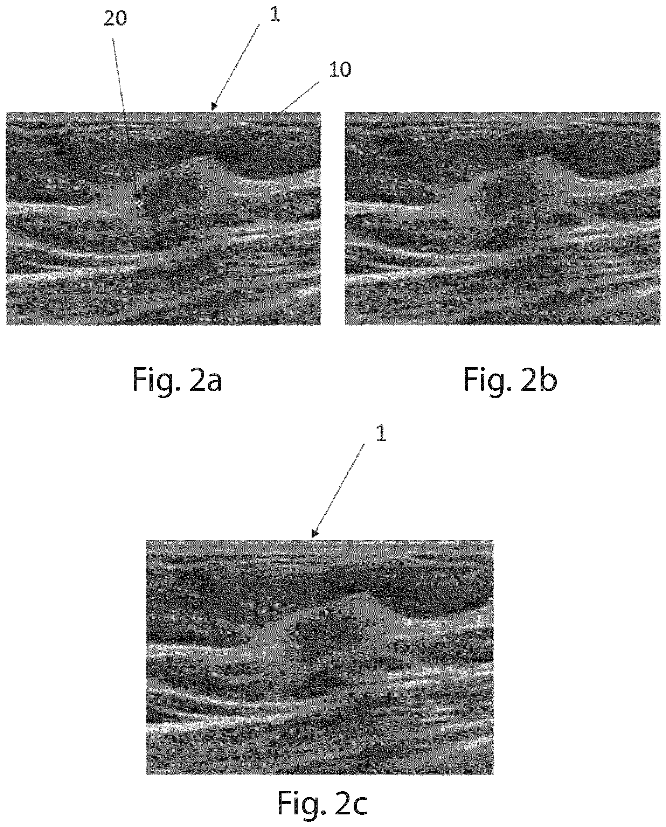

The ultrasound image 1 showing the primary tumor diagnosed by the radiologist acquired in step 101 may contain some markers 20 as shown for example in FIG. 2a.

In particular, during the screening step and after having identified the primary tumor 10, the radiologist can position some markers 20 on the ultrasound image 1 to delimit the region of interest containing the primary tumor 10. Therefore, the pixels of the original image corresponding to these markers 20 can be compromised.

The method 100 may comprise a step in which it is determined whether said ultrasound image 1 comprises one or more markers 20, wherein each of said one or more markers 20 is associated with one or more pixels of said ultrasound image 1.

The method 100 may further comprise a step in which each of said one or more markers 20 is removed and the one or more corresponding pixels are restored 102 before proceeding with the segmentation step 103 of the ultrasound image 1.

This step allows to remove the compromised pixels and to replace them by using, for example, an *exemplar-based* synthesis of the image, i.e., by generating a new image which is perceptually similar to the ultrasound image 1 obtained from the ultrasound examination.

In more details, the step of removing and restoring the pixels of the image may comprise the steps of:

A1. Associating the indicator I to the numerical array that represents the ultrasound image 1, verifying whether I represents a numerical array of size m-by-n-by-3, thus a *Truecolor* image, or a numerical array m-by-n that encodes a grayscale image.
If the image is a *Truecolor* image, it can be converted to grayscale. The conversion operation may, for example, be performed by using the Matlab® function *rgb2gray* that executes, pixels by pixel, a weighted sum of the RGB components as follows:

$$0.2989 * R + 0.5870 * G + 0.1140 * B.$$

A2. Obtaining the image J showing the marker 20 and detecting the marker 20 on the ultrasound image 1. For this purpose, the following procedure is implemented:

i. calculating the normalized cross-correlation in the spatial domain between the two matrices of the input image I and of the image J, using the Matlab® *normxcorr2:* this operation makes it possible to identify geographically related pixels in the two images.

ii. finding the coordinates (xpeak1, ypeak1) and (xpeak2, ypeak2) of the two peaks of the cross-correlation matrix calculated in step (i): the two peaks correspond to the final points of two markers 20 in I.

iii. identifying the final points of the other two markers 20 by adding the two dimensions of J to the coordinates (xpeak1, ypeak1) and (xpeak2, ypeak2).

iv. drawing a rectangle delimiting the corresponding area (FIG. 2b) using the four final points calculated in steps (ii) and (iii).

v performing the inpaint process utilizing the Matlab® *inpaintCoherent* function. Starting from the boundary pixels of the identified rectangle, generating a binary mask of the same size as the input image I where the pixels other than zero corresponds to the region to be designed. Thus, for each pixel in this region, computing the inpainted value as a weighted average of the known pixel values within the radius of inpainting. The weighted averages are assigned as follows: after estimating the coherence direction d, the pixels along d are associated with a higher average value than the pixels along other directions. Thus, the compromised pixel values are replaced with the corresponding calculated average values, obtaining an ultrasound image 1 without markers as shown, for example, in FIG. 2c.

With reference to FIG. 1, the method 100 comprises the segmentation step 103 of the ultrasound image. The segmentation stage 103 enables the region of interest (ROI) to be correctly identified, thus avoiding false candidates.

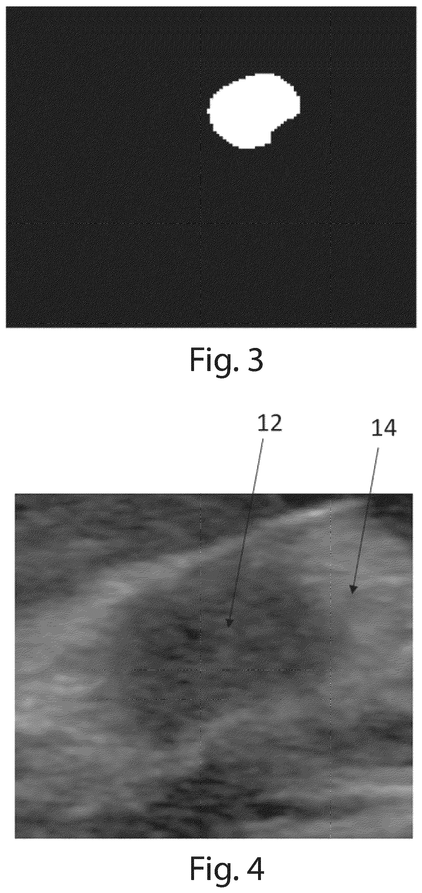

Step 103 comprises determining a ROI comprising an intratumoral region 12 of the primary tumor 10 and a peritumoral region 14 of the primary tumor 10 as shown, for example, in FIG. 4.

The segmentation step 103 includes performing a semantic segmentation process using a CNN that was trained to determine said intratumoral region 12 of the primary tumor 10.

The ROI determination step may further include a step of delineating the intratumoral region 12 of the primary tumor 10 and the peritumoral region 14 of the primary tumor 10.

As described below, the semantic segmentation process may utilize a *Xception* architecture such as a CNN pre-trained. The training may include a learning step using over three thousand ultrasound images acquired, for example, by seven ultrasonic devices. Said segmenting process is described in Wilfrido Gómez-Flores, Wagner Coelho de Albuquerque Pereira, "A comparative study of pre-trained convolutional neural networks for semantic segmentation of breast tumors in ultrasound", Computers in Biology and Medicine, Volume 126, 2020, 104036, ISSN 0010-4825, (https://www.sciencedirect.com/science/article/pii/S001048252030367X).

The segmentation step 103 described above may comprise the following sub-steps:

B1. Associating the indicator I* to the numerical array that represents the ultrasound image 1 and resizing the ultrasound image 1 to obtain an image of dimensions 128×128 pixels, as required by the CNN *Xception* architecture used.

B2. Implement a semantic segmentation procedure utilizing Matlab® *semanticseg* function. This function allows to classify each pixel in I*, assigning the label tumor/normal to each pixel. For each pixel, the assigned label can be subsequently allocated in a semantic array S of the same size as the input ultrasound image 1 of input I*.

B3. Resizing both the image I* and the semantic array S to the original dimension of the ultrasound image 1.

B4. Delineating both the identified intratumoral region 12 and a peritumoral region 14 which could store information relevant for the estimation of the status of the sentinel lymph node, since it comprises the connecting tissue between the primary tumor 10 and normal tissue, and thus represents an area of tumor proliferation and angiogenesis.

The delineation step can comprise the following sub-steps:

i. defining a binary mask of the same size as the input image I*, assigning an intensity value 0 to the pixels that have been designated as normal and an intensity value 1 to the pixels that have been designate as cancer (FIG. 3). The region identified by the pixels designated as cancer defines the intratumoral region.

ii. determining boundary pixels p1, p2, p3 and p4 of said intratumoral region on the binary mask.

iii. drawing a rectangle whose perimeter pixels are determined by adding a number n of proximal pixels to each boundary pixel p1, p2, p3 and p4 detected in step (ii) in order to add the intratumoral region to the peritumoral region.

iv. delineating the rectangle identified in the previous step on the input image I* to extract the ROI comprising both the intratumoral region 12 and the peritumoral region 14 (FIG. 4).

The segmentation stage 103 of the ultrasound image 1 also comprises the extraction of a plurality of features from said ultrasound image 1.

In an embodiment, such extraction can be performed using the dec_relu4 level of the Xception architecture, which corresponds to the last activation layer of the architecture Xception before classification by means of such an architecture. In particular, the dec_relu4 level is an activation level which takes as input the convolutional layer output at the end of the network. This level has an output of size 32x32x256 corresponding to a single length vector 262144, representing the number of features extracted using the Xception architecture. Since the dec_relu4 level is one of the final levels of the Xception architecture, the extracted features corresponding to this level are features of high level, i.e., global details of the ultrasound image 1, such as, for example, tissue and morphological characteristics of the entire ultrasound image 1, which may comprise, for example, shapes and/or objects.

With reference to FIG. 1, the method 100 comprises the step of extracting 105 a plurality of characteristics of said ultrasound image 1 from said ROI using a *transfer learning* approach, according to which a pre-developed model for performing a given activity is used as a starting point to develop a new model that performs a different activity. The transfer learning approach is described in Pan, Sinno Jialin; Yang, Qiang (2010). "A Survey on Transfer Learning". IEEE Transactions on Knowledge and Data Engineering, 22(10), 1345-1359. doi:10.1109/TKDE.2009.191. Such an approach allows to use a convolutional neural network previously trained on a large number of images of any type in order to be able to perform automatic extraction of features on different levels of abstraction, including both low-level features, e.g., edges, points and/or curves, and high-level features, e.g., shapes and/or objects, from an image. For example, the characteristic extraction step can be implemented by a pre-trained CNN architecture, such as the AlexNET architecture.

The extraction step 105 of a plurality of characteristics may comprise the following sub-steps:

C1. associating the indicator R with a numerical array that represents the ROI previously determined, resizing the image of the ROI to obtain an image of size $227 \times 227$ pixel, as required by the AlexNET architecture.
C2. extracting features of the re-sized ROI image from the pool2 level of the AlexNet architecture that corresponds to the second pooling level after the second convolutional layer. The pool2 level has an output of size $13 \times 13 \times 256$ corresponding to a single vector of length 43264 units. As a consequence, the number of features extracted is 43264 in total. Since the pool2 level is one of the initial levels of the network, the extracted features corresponding to this level are low-level features, i.e., representations of local details of an image, e.g., edges, points and/or curves.

The features extracted from the ultrasound image in the segmentation stage 103, for example by using the dec_relu4 level of the Xception architecture, and the respective ROI in the extraction step 105 are aggregated in a single vector comprising said characteristics. Such an aggregation comprises juxtapositioning in said vector said one or more features extracted in step 105 to said further plurality of features extracted in step 103 from said entire ultrasound image 1.

The method 100 further comprises the step of selecting 106 the one or more characteristics from the plurality of characteristics of the image. The selection step 106 comprises selecting a plurality of features stored in said vector, wherein said plurality of selected features comprises one or more characteristics selected from said plurality of characteristics extracted from said ROI and one or more features selected from said further plurality of features extracted from said entire ultrasound image. The selection step 106 of said one or more characteristics may comprise performing one or more validation cycles to estimate a predictive power score for each of said plurality of characteristics of said image and/or said additional plurality of features extracted in step 103, wherein performing one or more validation cycles may comprise, for each cycle, performing the steps of:

a) for each of the plurality of characteristics/features, calculate an area, AUC, below a receiver operational characteristic curve, ROC, associated with said characteristic of said image and estimate said predictive power score of said image characteristic based on said AUC;
b) for each of the plurality of features, comparing said predictive power score with a selected threshold value, and in response to a determination that the predictive power score reaches said selected value, associating the image characteristic to said one or more selected features.

The selection step 106 allows to reduce the size of the set of original characteristics comprising the plurality of features extracted in step 105 of the method by identifying one or more relevant characteristics for estimating the status of the sentinel lymph node. These features are more correlated with the metastatic nodal status. Through the use of these characteristics it is possible to more accurately determine whether the primary tumor 10 has acquired the ability to migrate to the sentinel lymph node, or that has migrated, thus obtaining a more accurate estimate of the metastatic status of the sentinel lymph node.

The selection step 106 makes it possible to further increase the predictive power of the algorithm by selecting the most critical variables and eliminating those redundant and irrelevant variables for the estimation of the metastatic status.

The implementation of a selection process also makes it possible to reduce the overfitting. Moreover, the presence of one or more non-redundant features implies smaller possibilities that the noise influences the final predictions.

The selection step 106 may be performed in a Matlab® environment and may include the following sub-steps: E1. implementing 5 cycles of an exhaustive one-layered random sampling procedure in 15 partitions to evaluate the discriminant power of each feature in predicting the status of the sentinel lymph node in the ultrasound images using the selected sub-group of images of the selected partitions. For this purpose, the following procedure may be implemented for each cycle:

i. splitting randomly and in a layered manner relative to the variable to predict the set of images in 15 partitions such that, in turn, a partition is excluded and said AUC is calculated using the images of the remaining 14 partitions, so that each image is used 14 times to test the predictive power of the single feature.

The layered aspect of the sampling enables to divide the set of images into homogeneous subpopulations with respect to the variable to be predicted, thus the percentage of patients with the metastatic lymph node is met in each partition.

The exhaustive aspect of the sampling enables the entire dataset of ultrasound images relative to the patients to be used, thus each partition is a good representing the dataset.

ii. for each cycle, computing the Area Under the Curve (AUC) of the Receiver Operating Characteristic (ROC) curve associated with each individual feature, utilizing the Matlab ® *perfcurve* function.

The ROC curve is a graph showing the performance that a classification model reaches in assigning a positive or negative label to each image, as the threshold varies. Thus, the AUC provides an aggregated measure of performance for all the considered classification thresholds.

iii. for each cycle, selecting only features associated with an AUC value greater than or equal to a threshold value of 70%.

E2. defining the new feature set comprising one or more features selected at least 70% of the times in the 5 cycles.

With reference to FIG. 1, the method 100 further comprises performing the classification step 107 of said status of the sentinel lymph node based on one or more characteristics of said plurality of characteristics of the image selected in step 106.

The classification step 107 is performed on the basis of the features stored within the vector, and therefore it is based on the features extracted in step 105 and of the features extracted from the entire ultrasound image during the segmentation step 103. This classification allows for a more exhaustive evaluation of the metastatic status of a sentinel lymph node.

The classification step 107 comprises:

training a machine learning model to predict the status of the sentinel lymph node based on the one or more selected characteristics; the model returns a probability of positivity of the status of the sentinel lymph node.

comparing said probability with a corresponding second threshold value to estimate the metastatic status of the sentinel lymph node. The metastatic status may comprise a positive metastatic status or a negative metastatic status of said sentinel lymph node.

The classification step 107 makes it possible to discriminate between patients characterized by a positive metastatic status of the lymph node and patients whose status of the sentinel lymph node is found to be non-metastatic, and thus negative, by assigning the positive/negative label to each of the images examined. The assessment of the metastatic status of the sentinel lymph node, which is the closest to the primary tumor and the first to be reached by the tumor cells that could be migrated from the primary tumor, is an explanatory and exhaustive evaluation of the metastatic status of the surrounding lymph nodes. The real metastatic status of the sentinel lymph node of the analyzed patients was retrospectively obtained as the result of the biopsy of the sentinel lymph node, which is performed to patients with breast cancer and clinically negative lymph nodes according to the clinical practice.

To this purpose, a Support Vector Machine (SVM) classifier may be used. The SVM classifier is a supervised machine learning model that identifies, among all possible hyperplanes separating two classes of data points, the one that has the maximum margin, i.e., the maximum distance between the data points from both classes, by means of a kernel function. The classification step 107 that uses the SVM classifier may comprise the following sub-steps:

F1. training the SVM classifier by means of the Matlab® function, specifying the following input parameters: 'Standardize' as 'true', 'KernelFunction' as 'linear', 'Kernelscale' as 'auto'. Thus, estimating the likelihood that an observation is classified as negative or positive by using the *fitPosterior* function in Matlab®.

Classify the observations using the *predict* function in Matlab® that calculates the probability that an observation belongs to a selected reference class.

F2. The estimated probabilities for each image are then compared with a predefined threshold value. The threshold value may be preset as the ratio between the number of images of the sentinel lymph node with positive metastatic status and the total number of available images. The images associated with a probability higher than the threshold value are assigned to the positive class. On the contrary, images characterized by a probability of less than or equal to the threshold value are assigned to the negative class.

The above-described method 100 allows to obtain a diagnostic accuracy level that is sufficient to support radiologists when evaluating the status of the sentinel lymph node.

The above-described method 100 was evaluated during validation phase, by testing the predictive ability of the method 100 on further images. The validation process may include the following sub-steps:

G1. training the SVM classifier by using of the Matlab® function *fitcsvm* by considering all the images in the training set and specifying the following input parameters: 'Standardize' as 'true', 'KernelFunction' as 'linear', 'Kernelscale' as 'auto'. Estimating the probability that an observation is classified as negative or positive by using the *fitPosterior* function of Matlab®.

G2. classifying all observations in the test set using the Matlab® function *predict* that calculates the probability that an observation belongs to a class.

G3. comparing the estimated probabilities for each test set image with the threshold value used in the classification step 107.

G4. evaluating the performance of the designed model in terms of Area Under the Curve (AUC) of the Receiver Operating Characteristic (ROC) curve and other standard metrics such as accuracy, sensitivity and specificity defined as follows:

$$\text{SENSITIVITY} = \frac{TP}{TP+FN} \qquad \text{SPECIFICITY} = \frac{TN}{TN+FP} \qquad \text{ACCURACY} = \frac{TP+TN}{TN+FP+TP+FN}$$

Whereby the variable TP indicates the number of patients correctly classified as positive, the TN variable indicates the number of patients correctly classified as negative, the variable FP indicates the number of negative patients erroneously classified as positive and the variable FN indicates the number of positive patients erroneously classified as negative.

The method described above allows to obtain performance with accuracy of 90.6%, sensitivity equal to 100% and specificity equal to 88.5%.

The method described above may be performed by one or more processors that have the task of coordinating the actions needed for the execution of one or more machine instruction relating to one or more processes, passages, or steps previously described.

In some embodiments, the one or more processors may be configured to generate control information, e.g., a report related to the estimated status of the sentinel lymph node, or respond to the received information, e.g., control information from a network node.

Said one or more processors may be part of a processing system configured to classify a status of a sentinel lymph node that may also include other auxiliary components, such as a random access memory (RAM) and a read-only memory (ROM). The processing system may include a memory, such as a storage medium readable by the one or more processors.

Examples of memory include volatile and nonvolatile memory, magnetic media, optical media, random access memory (RAM), read-only memory (ROM), removable media and others. The memory may be a computer program product comprising one or more instructions that when executed by one or more processors, causes a system to perform processes, passages, or steps of the method previously described.

Advantageously, by means of the method, object of the present invention, it is possible to predict the status of a sentinel lymph node of patients with breast carcinoma in a more accurate manner by exploiting only the characteristics of various nature automatically extracted from the primary tumor ultrasound images acquired for example during the diagnosis phase.

The features extracted from the entire ultrasound image in the segmentation step 103 and those extracted in step 105 represent complementary information, which allow for a more exhaustive evaluation of the metastatic status of a sentinel lymph node. The features extracted from the entire ultrasound image in the segmentation stage 103 therefore include additional information that does not overlap with those extracted from the ROI, since they describe features of the whole image and therefore can be juxtaposed for the purposes of classification without creating redundancy. Furthermore, the depth and sensitivity levels with which such features are identified and extracted by the method described above allow to define more accurate predictive models which, importantly, are not dependent on the operators. In fact, the method according to the present invention represents a fully automated instrument starting from the acquisition of ultrasound images.

A second advantage of the method subject of the present invention is to predict the status of a sentinel lymph node of patients affected by breast carcinoma in a more robust manner since the method is based on machine learning and deep learning techniques. Such neural networks are capable of more accurately segmenting the image by properly localizing the intratumoral region 12 of the primary tumor 10 and detecting textural and morphological characteristics of the entire ultrasound image 1 and details of the ROI which are not easily identifiable by an operator thus providing a more efficient solution.

Therefore, the method described above is an accurate and non-invasive approach for the prediction of the lymphatic status in patients affected by breast cancer with clinically negative lymph nodes.

A further advantage is that the described method represents a less invasive and expensive technique with respect to other diagnostic tools. Therefore, the disclosed invention could support radiologists when estimating the status of the sentinel lymph node in a simple and accurate manner.

The present invention has been described for illustrative but not limitative purposes, according to its preferred embodiments, but it is appreciated that variations and/or modifications may be made by those skilled in the art without departing from the relative scope of protection, as defined by the appended claims.

## Claims

1.   A computer-implemented method (100) for estimating the metastatic status of a sentinel lymph node based on an ultrasound image (1) of the primary tumor (10),

   the method (100) comprising:

      segmenting (103) said ultrasound image (1), wherein segmenting said ultrasound image (1) includes determining a region of interest, ROI, comprising an intratumoral region (12) of the primary tumor (10) and a peritumoral region (14) of the primary tumor (10), and wherein segmenting (103) said ultrasound image (1) comprises:

         performing a semantic segmentation process using a first convolutional neural network, CNN, that has been trained to determine said intratumoral region (12) of the primary tumor (10); and
         extracting a plurality of features from said entire ultrasound image (1) by means of and within layers of said first CNN, said features being high-level features, preferably wherein said features comprise at least one of a shape and an object;

      extracting (105) a plurality of characteristics of said image from said ROI using a transfer learning approach employing a pre-trained CNN that is different than said first CNN;
      aggregating into a vector said plurality of characteristics extracted from said ROI and said plurality of features extracted from said entire ultrasound image (1); and
      selecting (106) a plurality of characteristics stored in said vector, wherein said plurality of selected characteristics comprises one or more

      characteristics selected from said plurality of characteristics extracted from said ROI and one or more features selected from said plurality of features extracted from said entire ultrasound image; and
      classifying (107) the metastatic status of said sentinel lymph node based on said plurality of characteristic selected from said vector.

2.   The method (100) according to claim 1, comprising:

      determining that the ultrasound image (1) includes one or more markers (20), wherein each of said one or more markers (20) is associated with one or more pixels of the ultrasound image (1);
      for each of said one or more markers (20), removing the marker (20) and reconstructing (102) said one or more pixels before segmenting (103) said ultrasound image (1).

3.   The method (100) of claim 2, wherein reconstructing (102) said one or more pixels includes performing an inpainting procedure.

4.   The method (100) of any one of the preceding claims, wherein determining a ROI comprises delineating said intratumoral region (12) of the primary tumor (10) and said peritumor region (14) of the primary tumor (10).

5. The method (100) of any one the preceding claims, wherein extracting (105) a plurality of characteristics of said image from said ROI includes extracting image features including low-level features, wherein said low-level features comprise representations of local details of said ROI.

6. The method (100) of claim 5, wherein said low-level characteristics include at least one of an edge, a point and a curve.

7. The method (100) of any one of the preceding claims, wherein extracting (105) a plurality of characteristics of said image from said ROI comprises extracting image features including high-level features, preferably wherein said high-level features include at least one of a shape and an object.

8. The method (100) of any one of the preceding claims, wherein selecting (106) said one or more characteristics includes:

   performing one or more evaluation cycles to estimate a predictive power score for each of said plurality of characteristics of said image;
   for each of said plurality of characteristics, comparing said predictive power score with a value selected from a plurality of threshold values, and in response to a determination that the predictive power score reaches the selected value, associating the image characteristic with said one or more characteristics.

9. The method (100) of claim 8 wherein performing one or more evaluation cycles includes, for each cycle and for each of said pluralities of threshold values, performing the steps of:
   for each of said pluralities of characteristics, calculating an area, AUC, below a receiver operational characteristic curve, ROC, associated with said characteristic of said image and estimate said predictive power score of said image characteristic based on said AUC.

10. The method (100) of any one of the preceding claims, wherein classifying (107) the metastatic status of said sentinel lymph node according to said one or more characteristics comprises:

    obtaining a probability that said sentinel lymph node corresponds to a metastatic status based on said one or more selected features; and
    comparing said probability with a corresponding second threshold value to estimate the metastatic status of the sentinel lymph node (10).

11. The method (100) of claim 10, wherein said metastatic status is a positive metastatic status or a negative metastatic status of said sentinel lymph node.

12. A system configured to classify a metastatic status of a sentinel lymph node, the system comprising:

    a memory; and
    one or more processors configured to execute the method of any one of the preceding claims.

13. A computer program product comprising one or more instructions that, when executed by one or more processors, cause the one or more processors to execute the method of any one of claims 1 to 11.


**Patentansprüche**

1. Computerimplementiertes Verfahren (100) zur Abschätzung des Metastasierungsstatus eines Sentinel-Lymphknotens auf der Grundlage einer Ultraschalluntersuchung Abbildung (1) des Primärtumors (10),
   das Verfahren (100) umfasst:
   Segmentierung (103) des genannten Ultraschallbildes (1), wobei die Segmentierung des genannten Ultraschallbildes (1) die Bestimmung eines Untersuchungsbereichs, ROI umfasst, der einen intratumoralen Bereich (12) des Primärtumors (10) und einen peritumoralen Bereich (14) des Primärtumors (10) umfasst, und wobei die Segmentierung (103) Das genannte Ultraschallbild (1) umfasst:

   Durchführung eines semantischen Segmentierungsprozesses unter Verwendung eines ersten konvolutionellen neuronalen Netzes, CNN, das darauf trainiert wurde, den genannten intratumoralen Bereich (12) des Primärtumors (10) zu bestimmen; und Extrahieren einer Vielzahl von Merkmalen aus dem gesamten Ultraschallbild Bild (1) mittels und innerhalb der Schichten des ersten CNN, wobei das wobei es sich um übergeordnete Merkmale handelt, vorzugsweise wobei die genannten Merkmale, die mindestens entweder eine Form oder ein Objekt umfassen; Extrahieren (105) einer Vielzahl von Merkmalen des Bildes aus dem ROI unter Verwendung eines Transfer-Learning-Ansatzes, bei dem ein vortrainiertes CNN zum Einsatz kommt, das sich von dem ersten CNN unterscheidet;

die aus dem ROI extrahierte Vielzahl von Merkmalen und die eine Vielzahl von Merkmalen, die aus dem gesamten Ultraschallbild (1) extrahiert wurden; und

Auswählen (106) einer Vielzahl von Merkmalen, die in dem Vektor gespeichert sind, wobei die Vielzahl ausgewählter Merkmale umfasst ein oder mehrere Merkmale, die aus der Vielzahl der aus dem ROI extrahierten Merkmale ausgewählt wurden, sowie ein oder mehrere Merkmale, die aus der eine Vielzahl von Merkmalen, die aus dem gesamten Ultraschallbild extrahiert wurden; und

Klassifizieren (107) des Metastasierungsstatus des Sentinel-Lymphknotens basierend auf der genannten Vielzahl von Merkmalen, die aus dem genannten Vektor ausgewählt wurden.

2.  Verfahren (100) nach Anspruch 1, umfassend:

Feststellen, dass das Ultraschallbild (1) einen oder mehrere Marker (20) enthält, wobei jeder der einen oder mehreren Marker (20) einem oder mehreren Pixeln des Ultraschallbildes (1) zugeordnet ist;
für jeden der einen oder mehreren Marker (20) das Entfernen des Markers (20) und das Rekonstruieren (102) des einen oder der mehreren Pixel vor dem Segmentieren (103) des Ultraschallbildes (1).

3.  Verfahren (100) nach Anspruch 2, wobei das Rekonstruieren (102) des einen oder der mehreren Pixel die Durchführung eines Inpainting-Verfahrens umfasst.

4.  Verfahren (100) nach einem der vorstehenden Ansprüche, wobei das Bestimmen eines ROI das Abgrenzen des intratumoralen Bereichs (12) des Primärtumors (10) und des peritumoralen Bereichs (14) des Primärtumors (10) umfasst.

5.  Verfahren (100) nach einem der vorstehenden Ansprüche, wobei das Extrahieren (105) einer Vielzahl von Merkmalen des Bildes aus dem ROI das Extrahieren von Bildmerkmalen umfasst, einschließlich Merkmalen niedriger Ebene, wobei die Merkmale niedriger Ebene Darstellungen lokaler Details des ROI umfassen.

6.  Verfahren (100) nach Anspruch 5, wobei die Low-Level-Merkmale mindestens eines der folgenden Elemente umfassen: eine Kante, einen Punkt und eine Kurve.

7.  Verfahren (100) nach einem der vorstehenden Ansprüche, wobei das Extrahieren (105) einer Vielzahl von Merkmalen des Bildes aus dem ROI das Extrahieren von Bildmerkmalen umfasst, einschließlich Merkmalen auf hoher Ebene, vorzugsweise, wobei die genannten übergeordneten Merkmale mindestens entweder eine Form oder ein Objekt umfassen.

8.  Verfahren (100) nach einem der vorstehenden Ansprüche, wobei das Auswählen (106) des einen oder der mehreren Merkmale Folgendes umfasst:

Durchführung eines oder mehrerer Auswertungszyklen zur Schätzung eines Vorhersagekraftwerts für jedes der zahlreichen Merkmale des Bildes;
für jedes der mehreren Merkmale den Vorhersagekraftwert mit einem Wert zu vergleichen, der aus einer Vielzahl von Schwellenwerten ausgewählt wurde, und als Reaktion auf die Feststellung, dass der Vorhersagekraftwert den ausgewählten Wert erreicht, das Bildmerkmal dem einen oder den mehreren Merkmalen zuzuordnen.

9.  Verfahren (100) nach Anspruch 8, wobei das Durchführen eines oder mehrerer Auswertungszyklen für jeden Zyklus und für jeden der mehreren Schwellenwerte die Durchführung der folgenden Schritte umfasst:
für jede der mehreren Merkmale die Fläche AUC unterhalb einer Empfänger-Operations-Charakteristik-Kurve ROC zu berechnen, die mit dem jeweiligen Merkmal des Bildes verbunden ist, und den Vorhersagekraft-Wert des Bildmerkmals auf der Grundlage der AUC zu schätzen.

10. Verfahren (100) nach einem der vorstehenden Ansprüche, wobei das Klassifizieren (107) des Metastasierungsstatus des Sentinel-Lymphknotens anhand des einen oder der mehreren Merkmale Folgendes umfasst:

Ermitteln einer Wahrscheinlichkeit, dass der genannte Sentinel-Lymphknoten einem Metastasierungsstatus entspricht, basierend auf dem einen oder den mehreren ausgewählten Merkmalen; und

Vergleich dieser Wahrscheinlichkeit mit einem entsprechenden zweiten Schwellenwert, um den Metastasierungsstatus des Sentinel-Lymphknotens (10) abzuschätzen.

11. Verfahren (100) nach Anspruch 10, wobei der Metastasierungsstatus ein positiver Metastasierungsstatus oder ein negativer Metastasierungsstatus des Sentinel-Lymphknotens ist.

12. System zur Bestimmung des Metastasierungsstatus eines Sentinel-Lymphknotens, wobei das System Folgendes umfasst:

eine Erinnerung; und

ein oder mehrere Prozessoren, die so konfiguriert sind, dass sie das Verfahren gemäß einem der vorstehenden Ansprüche ausführen.

13. Computerprogrammprodukt, das eine oder mehrere Anweisungen umfasst, die, wenn sie von einem oder mehreren Prozessoren ausgeführt werden, den einen oder die mehreren Prozessoren veranlassen, das Verfahren eines der Ansprüche 1 bis 11 auszuführen.

**Revendications**

1. Procédé mis en œuvre par ordinateur (100) permettant d'évaluer le statut métastatique d'un ganglion sentinelle à l'aide d'une échographie image (1) de la tumeur primaire (10),
le procédé (100) comprenant:
segmenter (103) ladite image échographique (1), la segmentation de ladite image échographique (1) comprenant la détermination d'une région d'intérêt, ROI, comprenant une région intratumorale (12) de la tumeur primaire (10) et une région péritumorale (14) de la tumeur primaire (10), et la segmentation (103) ladite image échographique (1) comprend:

en effectuant un processus de segmentation sémantique à l'aide d'un premier réseau neuronal convolutif, CNN, qui a été entraîné pour déterminer ladite région intratumorale (12) de la tumeur primaire (10); et
extraire plusieurs caractéristiques de l'ensemble de ladite image échographique image (1) au moyen et au sein des couches dudit premier réseau neuronal convolutif CNN, ledit ces caractéristiques étant des caractéristiques de haut niveau, de préférence dans lesquelles lesdites les caractéristiques comprennent au moins un élément parmi une forme et un objet;
extraire (105) une pluralité de caractéristiques de ladite image à partir de ladite région d'intérêt ROI à l'aide d'une approche d'apprentissage par transfert utilisant un réseau neuronal convolutif CNN pré-entraîné différent dudit premier CNN;
en regroupant en un vecteur ladite pluralité de caractéristiques extraites de ladite ROI zone d'intérêt et ladite une pluralité de caractéristiques extraites de ladite image échographique complète (1); et
sélectionner (106) une pluralité de caractéristiques stockées dans ledit vecteur, dans lequel ladite pluralité de caractéristiques sélectionnées comprend une ou plusieurs caractéristiques choisies parmi ladite pluralité de caractéristiques extraites de ladite région d'intérêt ROI et une ou plusieurs caractéristiques choisies parmi ladite une pluralité de caractéristiques extraites de ladite image échographique complète; et
la classification (107) du statut métastatique dudit ganglion lymphatique sentinelle sur la base de ladite pluralité de caractéristiques sélectionnées à partir dudit vecteur.

2. Procédé (100) selon la revendication 1, comprenant:

en déterminant que l'image échographique (1) comprend un ou plusieurs marqueurs (20), chacun desdits un ou plusieurs marqueurs (20) étant associé à un ou plusieurs pixels de l'image échographique (1);
pour chacun desdits un ou plusieurs marqueurs (20), retirer le marqueur (20) et reconstruire (102) lesdits un ou plusieurs pixels avant de segmenter (103) ladite image échographique (1).

3. Procédé (100) selon la revendication 2, dans lequel la reconstruction (102) dudit ou desdits pixels comprend la mise en œuvre d'une procédure de retouche.

4. Procédé (100) selon l'une quelconque des revendications précédentes, dans lequel la détermination d'une zone d'intérêt ROI comprend la délimitation de ladite région intratumorale (12) de la tumeur primaire (10) et de ladite région péritumorale (14) de la tumeur primaire (10).

5. Procédé (100) selon l'une quelconque des revendications précédentes, dans lequel l'extraction (105) d'une pluralité de caractéristiques de ladite image à partir de ladite région d'intérêt ROI comprend l'extraction de caractéristiques d'image, y compris des caractéristiques de bas niveau, lesdites caractéristiques de bas niveau comprenant des représentations de détails locaux de ladite région d'intérêt ROI.

6. Procédé (100) selon la revendication 5, dans lequel lesdites caractéristiques de bas niveau comprennent au moins l'un parmi un bord, un point et une courbe.

7. Procédé (100) selon l'une quelconque des revendications précédentes, dans lequel l'extraction (105) d'une pluralité de caractéristiques de ladite image à partir de ladite région d'intérêt ROI comprend l'extraction de caractéristiques d'image, y compris des caractéristiques de haut niveau, de préférence, lesdites caractéristiques de haut niveau comprenant au moins l'une parmi une forme et un objet.

8. Procédé (100) selon l'une quelconque des revendications précédentes, dans lequel la sélection (106) de ladite ou desdites caractéristiques comprend:

en effectuant un ou plusieurs cycles d'évaluation afin d'estimer un score de pouvoir prédictif pour chacune des caractéristiques de ladite image;
pour chacune desdites caractéristiques, comparer ledit score de pouvoir prédictif à une valeur choisie parmi une pluralité de valeurs seuils, et, dès lors qu'il est déterminé que le score de pouvoir prédictif atteint la valeur choisie, associer la caractéristique de l'image à ladite ou auxdites caractéristiques.

9. Procédé (100) selon la revendication 8, dans lequel l'exécution d'un ou plusieurs cycles d'évaluation comprend, pour chaque cycle et pour chacune desdites pluralités de valeurs seuils, l'exécution des étapes suivantes:
pour chacune desdites pluralités de caractéristiques, calculer une aire, AUC, sous une courbe caractéristique opérationnelle du récepteur, ROC, associée à ladite caractéristique de ladite image et estimer ledit score de pouvoir prédictif de ladite caractéristique de l'image sur la base de ladite AUC.

10. Procédé (100) selon l'une quelconque des revendications précédentes, dans lequel la classification (107) du statut métastatique dudit ganglion lymphatique sentinelle en fonction de ladite ou desdites caractéristiques comprend:

obtenir une probabilité que ledit ganglion sentinelle corresponde à un statut métastatique sur la base de ladite ou desdites caractéristiques sélectionnées; et
en comparant ladite probabilité à une deuxième valeur seuil correspondante afin d'évaluer le statut métastatique du ganglion sentinelle (10).

11. Procédé (100) selon la revendication 10, dans lequel ledit statut métastatique correspond à un statut métastatique positif ou à un statut métastatique négatif dudit ganglion sentinelle.

12. Système conçu pour déterminer le statut métastatique d'un ganglion sentinelle, ledit système comprenant:

un souvenir; et
un ou plusieurs processeurs configurés pour mettre en œuvre le procédé selon l'une quelconque des revendications précédentes.

13. Produit de programme informatique comprenant une ou plusieurs instructions qui, lorsqu'il est exécuté par un ou plusieurs processeurs, amènent le ou les processeurs à exécuter le procédé selon l'une quelconque des revendications 1 à 11.

<u>100</u>

| Acquire an ultrasound image of the primary tumor | 101 |

| Removing marker and reconstructing<br>pixels of the acquired ultrasound image | 102 |

| Automatically segmenting the ultrasound image:<br>determining a ROI comprising an intratumoral region<br>and a peritumoral region of the primary tumor | 103 |

| Extracting a plurality of characteristics from the ROI | 105 |

| Selecting a set of characteristics | 106 |

| Classifying the status of said sentinel lymph node | 107 |

# Fig. 1

Fig. 2a

Fig. 2b

Fig. 2c

Fig. 3

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BOVE, S.** ; **COMES, M.C.** ; **FANIZZI, A.** ; **MASSAFRA, R.** A ultrasound-based radiomic approach to predict the nodal status in clinically negative breast cancer patients. *Sci Rep*, 12 May 2022, vol. 12 (2022), 7914, https://doi.org/10.1038/s41598-022-11876-4
- **LEE YAN-WEI et al.** Axillary lymph node metastasis status prediction of early-stage breast cancer using convolutional neural networks. *COMPUTERS IN BIOLOGY AND MEDICINE*, 01 March 2021, vol. 130, 104206
- From Handcrafted to Deep-Learning-Based Cancer Radiomics: Challenges and opportunities. **AFSHAR PARNIAN et al.** IEEE SIGNAL PROCESSING MAGAZINE. IEEE, 01 July 2019, vol. 36, 132-160
- Feature selection using ROC curves on classification problems. **SERRANO A J et al.** NEURAL NETWORKS (IJCNN), THE 2010 INTERNATIONAL JOINT CONFERENCE ON. IEEE, 18 July 2010, 1-6
- **WILFRIDO GÓMEZ-FLORES** ; **WAGNER COELHO DE ALBUQUERQUE PEREIRA**. A comparative study of pre-trained convolutional neural networks for semantic segmentation of breast tumors in ultrasound. *Computers in Biology and Medicine*, 2020, vol. 126, ISSN 0010-4825, 104036, https://www.sciencedirect.com/science/article/-pii/S001048252030367X
- **PAN, SINNO JIALIN** ; **YANG, QIANG**. A Survey on Transfer Learning. *IEEE Transactions on Knowledge and Data Engineering*, 2010, vol. 22 (10), 1345-1359